# EUROPEAN PATENT APPLICATION

(11) **EP 4 410 823 A1**
(43) Date of publication of application: **07.08.2024**
(21) Application number: 22876420.5
(22) Date of filing: 29.09.2022
(51) Int. Cl.: C07K 14/315, A23K 10/16, A23K 20/147, A23K 50/80, C12N 1/00, C12N 1/20, C12N 15/31

(54) **FISH REARING COMPOSITION, AND COMPOSITION FOR TREATING OR PREVENTING FISH DISEASES**

(30) Priority: 30.09.2021 JP 2021161630
(71) Applicant: Sumitomo Chemical Company, Limited, Tokyo 103-6020 (JP)
(72) Inventor: AOKI, Mikio, Osaka-shi, Osaka 554-8558 (JP); MIKATA, Kazuki, Osaka-shi, Osaka 554-8558 (JP); KAI, Toshihiro, Osaka-shi, Osaka 554-8558 (JP); KUWAHARA, Hiroshi, Osaka-shi, Osaka 554-8558 (JP)
(74) Representative: Vossius & Partner Patentanwälte Rechtsanwälte mbB
(86) International application number: PCT/JP2022/036362
(87) International publication number: WO 2023/054554

(57) **Abstract**

Provided are: a fish rearing composition containing lacticin; and a fish rearing composition containing bacterial cells or a cell culture product of a bacterium having a lacticin gene, or an extract thereof. Provided is a method for rearing an organism of fish, including applying lacticin to the organism of fish, or applying bacterial cells or a cell culture product of a bacterium having a lacticin gene, or an extract thereof to the organism of fish. The lacticin is preferably lacticin Q.

## Description

### TECHNICAL FILED

The present invention relates to a fish rearing composition, and a composition for preventing or treating a fish disease. The present invention also relates to a method for rearing an organism of fish with the composition.

### BACKGROUND ART

*Lactococcus garvieae* infects organisms of fish and causes streptococcosis. Streptococcosis is a fish disease causing the largest amount of damage in the aquaculture of Japanese yellowtail. Conventionally, vaccine injection to juvenile fish has been performed in advance for the epidemic of streptococcosis. However, new-type pathogenic bacteria have appeared and are causing a problem of failure in preventing the onset of streptococcosis even with vaccine administration.

WO 2006/033352 (PTL 1) newly reports lacticin Q, which is an antibacterial peptide produced by lactic acid bacterium. WO 2017/069227 (PTL 2) discloses a healthcare composition including lacticin. CN103214583 (PTL 3) discloses a lacticin Q-SUMO fusion protein.

### CITATION LIST

### PATENT LITERATURE

PTL 1: WO 2006/033352
PTL 2: WO 2017/069227
PTL 3: CN103214583

### SUMMARY OF INVENTION

### TECHNICAL PROBLEM

An object of the present invention is to provide a novel composition and rearing method to inhibit fish diseases.

### SOLUTION TO PROBLEM

The present invention relates to items exemplified in the following.
[1] A composition for rearing an organism of fish, comprising at least one selected from the group consisting of lacticin, bacterial cells or a cell culture product of a bacterium having a lacticin gene, and an extract of the bacterial cells or the cell culture product.
[2] A composition for preventing or treating a disease caused by *Lactococcus garvieae* in fish, comprising at least one selected from the group consisting of lacticin, bacterial cells or a cell culture product of a bacterium having a lacticin gene, and an extract of the bacterial cells or the cell culture product.
[3] The composition according to [1] or [2], wherein the lacticin is a peptide containing any sequence of (a) to (c) and having antibacterial activity:
   (a) an amino acid sequence set forth in SEQ ID NO: 1,
   (b) an amino acid sequence having 69% or more identity with an amino acid sequence set forth in SEQ ID NO: 1, and
   (c) an amino acid sequence formed by deletion, substitution, insertion, and/or addition of 1 or more and 16 or less amino acid residues in an amino acid sequence set forth in SEQ ID NO: 1.
[4] The composition according to any of [1] to [3], wherein the lacticin is at least one selected from lacticin Q and lacticin Z, and the lacticin gene is at least one selected from a lacticin Q gene and a lacticin Z gene.
[5] The composition according to any of [1] to [4], wherein the bacterium having the lacticin gene is NITE BP-03536.
[6] The composition according to any of [1] to [5], wherein the fish belongs to the order Perciformes or the order Salmoniformes.
[7] The composition according to any of [1] to [6], wherein the composition is a feed, a feed additive, rearing water, a rearing water additive, or an injection.
[8] A method for rearing an organism of fish, comprising applying the composition according to any of [1] to [7] to the organism of fish.
[9] A method for preventing or treating a disease caused by *Lactococcus garvieae* in fish, comprising applying the composition according to any of [1] to [7] to the organism of fish.
[10] The method according to [8] or [9], wherein the applying is oral administration, soaking administration, or injection administration.
[11] The method according to any of [8] to [10], wherein the fish belongs to the order Perciformes or the order Salmoniformes.
[12] A growth inhibitor or a bactericide for *Lactococcus garvieae,* comprising at least one selected from the group consisting of lacticin, bacterial cells or a cell culture product of a bacterium having a lacticin gene, and an extract of the bacterial cells or the cell culture product.
[13] A growth-inhibiting or bactericidal method for *Lactococcus garvieae,* comprising bringing at least one selected from the group consisting of lacticin, bacterial cells or a cell culture product of a bacterium having a lacticin gene, and an extract of the bacterial cells or the cell culture product into contact with *Lactococcus garvieae.*
[14] A bacterium of Accession Number: NITE BP-03536.
[15] Bacterial cells or a cell culture product of the bacterium according to [14], or an extract thereof.
[16] A method for rearing an organism of fish, comprising applying at least one selected from the group consisting of lacticin, bacterial cells or a cell culture product of a bacterium having a lacticin gene, and an extract of the bacterial cells or the cell culture product to an organism of fish.
[17] A method for preventing or treating a disease caused by *Lactococcus garvieae* in fish, comprising applying at least one selected from the group consisting of lacticin, bacterial cells or a cell culture product of a bacterium having a lacticin gene, and an extract of the bacterial cells or the cell culture product to an organism of fish.
[18] The method according to [16] or [17], wherein the lacticin is a peptide containing any sequence of (a) to (c) and having antibacterial activity:
   (a) an amino acid sequence set forth in SEQ ID NO: 1,
   (b) an amino acid sequence having 69% or more identity with an amino acid sequence set forth in SEQ ID NO: 1, and
   (c) an amino acid sequence formed by deletion, substitution, insertion, and/or addition of 1 or more and 16 or less amino acid residues in an amino acid sequence set forth in SEQ ID NO: 1.
[19] The method according to any of [16] to [18], wherein the lacticin is at least one selected from lacticin Q and lacticin Z, and the lacticin gene is at least one selected from a lacticin Q gene and a lacticin Z gene.
[20] The method according to any of [16] to [19], wherein the bacterium having the lacticin gene is NITE BP-03536.
[21] The method according to any of [16] to [20], wherein the applying is oral administration, soaking administration, or injection administration.
[22] The method according to any of [16] to [21], wherein the fish belongs to the order Perciformes or the order Salmoniformes.

### ADVANTAGEOUS EFFECTS OF INVENTION

The present invention can provide a novel composition and rearing method to inhibit fish diseases.

### BRIEF DESCRIPTION OF DRAWINGS

Fig. 1 shows a result of homology analysis between the amino acid sequence set forth in SEQ ID NO: 1 and an amino acid sequence of lacticin Z.
Fig. 2 shows a result of homology analysis between the amino acid sequence set forth in SEQ ID NO: 1 and an amino acid sequence of an aureocin A53 family (WP_123311236.1).
Fig. 3 shows photographs showing (A) the colony shape of and (B) a result of Gram staining of NITE BP-03536 in Experiment 1.
Fig. 4 shows a diagram illustrating an antibacterial activity test of NITE BP-03536 in Experiment 2.
Fig. 5 shows a graph illustrating the number of surviving Japanese yellowtails exposed to *Lactococcus garvieae* in Experiment 3.

### DESCRIPTION OF EMBODIMENTS

Hereinafter, modes of implementation of the present invention will be described in detail. The present invention is not limited to the following embodiments. Herein, each expression in the format "A to B" indicates the upper limit and lower limit of a range (i.e., A or more and B or less), and if a unit is shown only for B but not for A, the units of A and B are the same.

### [Compositions for rearing organism of fish]

A composition for rearing an organism of fish according to an embodiment of the present invention contains at least one selected from the group consisting of lacticin, bacterial cells or a cell culture product of a bacterium having a lacticin gene, and an extract of the bacterial cells or the cell culture product of the bacterium having the lacticin gene. The compositions for rearing an organism of fish according the present invention can inhibit streptococcosis caused by *Lactococcus garvieae* in fish.

The lacticin may be at least one selected from lacticin Q and lacticin Z. The lacticin is preferably lacticin Q.

Herein, the lacticin may be a peptide containing any sequence of (a) to (c) below. The lacticin may have antibacterial activity to *Lactococcus garvieae.*
(a) An amino acid sequence set forth in SEQ ID NO: 1,
(b) an amino acid sequence having 69% or more identity with an amino acid sequence set forth in SEQ ID NO: 1, and
(c) an amino acid sequence formed by deletion, substitution, insertion, and/or addition of 1 or more and 16 or less amino acid residues in an amino acid sequence set forth in SEQ ID NO: 1.

The lacticin may be a peptide containing an amino acid sequence having, for example, 70% or more, 75% or more, 80% or more, 85% or more, 90% or more, 95% or more, 96% or more, 97% or more, 98% or more, or 99% or more identity with the amino acid sequence set forth in SEQ ID NO: 1. The upper limit of the identity of the amino acid sequence is not limited, and the identity may be, for example, 100% or less.

The lacticin may be a peptide containing an amino acid sequence formed by deletion, substitution, insertion, and/or addition of, for example, 1 or more and 15 or less, 14 or less, 13 or less, 12 or less, 11 or less, 10 or less, 9 or less, 8 or less, 7 or less, 6 or less, 5 or less, 4 or less, 3 or less, or 2 or less amino acid residues in the amino acid sequence set forth in SEQ ID NO: 1.

An example of substitution, deletion, insertion, and/or addition of an amino acid residue is conservative mutation, through which normal protein functions are maintained. A representative example of conservative mutation is conservative substitution. Conservative substitution is mutation to cause mutual substitution among Phe, Trp, and Tyr for aromatic amino acids at the site of substitution, among Leu, Ile, and Val for hydrophobic amino acids at the site of substitution, between Gln and Asn for polar amino acids, among Lys, Arg, and His for basic amino acids, between Asp and Glu for acidic amino acids, and between Ser and Thr for amino acids having a hydroxyl group. Examples of substitution regarded as conservative substitution include substitution of Ala with Ser or Thr, substitution of Arg with Gln, His, or Lys, substitution of Asn with Glu, Gln, Lys, His, or Asp, substitution of Asp with Asn, Glu, or Gln, substitution of Cys with Ser or Ala, substitution of Gln with Asn, Glu, Lys, His, Asp, or Arg, substitution of Glu with Gly, Asn, Gln, Lys, or Asp, substitution of Gly with Pro, substitution of His with Asn, Lys, Gln, Arg, or Tyr, substitution of Ile with Leu, Met, Val, or Phe, substitution of Leu with Ile, Met, Val, or Phe, substitution of Lys with Asn, Glu, Gln, His, or Arg, substitution of Met with Ile, Leu, Val, or Phe, substitution of Phe with Trp, Tyr, Met, Ile, or Leu, substitution of Ser with Thr or Ala, substitution of Thr with Ser or Ala, substitution of Trp with Phe or Tyr, substitution of Tyr with His, Phe, or Trp, and substitution of Val with Met, Ile, or Leu.

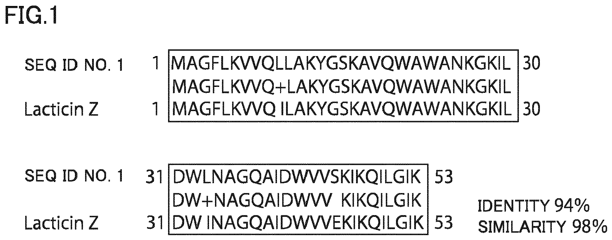

The lacticin may be a peptide consisting of an amino acid sequence set forth in SEQ ID NO: 1 or 3. SEQ ID NO: 1 shows an amino acid sequence of lacticin Q, which is predicted from the genome sequence of NITE BP-03536. SEQ ID NO: 3 shows an amino acid sequence of lacticin Z, which is predicted from the genome sequence of *Lactococcus lactis.* The identity and the similarity (positive) between the amino acid sequence set forth in SEQ ID NO: 1 and the amino acid sequence of lacticin Z are 94% and 98%, respectively (Fig. 1). There are three amino acid residue substitutions between the amino acid sequence set forth in SEQ ID NO: 1 and the amino acid sequence of lacticin Z, two of which are conservative substitutions. Lacticin Z is highly homologous to lacticin Q.

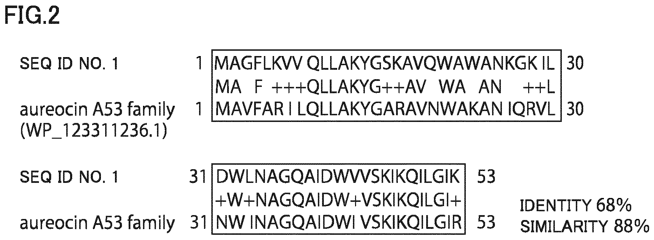

According to the results from homology search, a known peptide that is the second highest homologous to lacticin Q after lacticin Z is an aureocin A53 family (WP_123311236.1). An amino acid sequence of aureocin A53 is shown in SEQ ID NO: 4. The identity and the similarity between the amino acid sequence set forth in SEQ ID NO: 1 and the amino acid sequence of the aureocin A53 family (WP_123311236.1) are 68% and 88%, respectively (Fig. 2). There are 17 amino acid residue substitutions between the amino acid sequence set forth in SEQ ID NO: 1 and the amino acid sequence of the aureocin A53 family (WP_123311236.1), 11 of which are conservative substitutions.

The methionine on the N-terminus of the amino acid sequence of the lacticin is usually formylated (N-formyl methionine). The formylated methionine may further be oxidized.

The lacticin may be a chemically synthesized one, or one produced in a microorganism having a lacticin gene. In an aspect of the present embodiment, the lacticin may be one synthesized by using a technique of organic synthesis, or one synthesized by using a gene engineering technique. The lacticin gene may be endogenous or exogenous.

The lacticin gene may be a gene that encodes the lacticin described above, or may be at least one selected from a lacticin Q gene and a lacticin Z gene. The lacticin gene is preferably the lacticin Q gene.

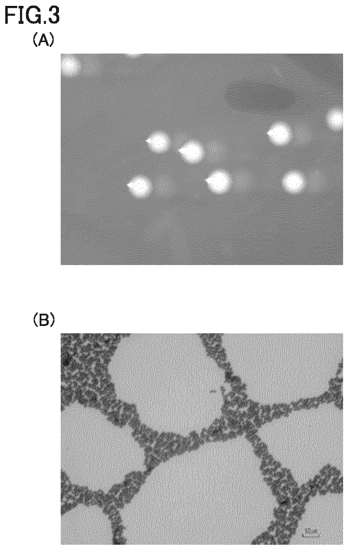

Examples of bacteria having a lacticin gene include NITE BP-03536. NITE BP-03536 is a bacterium internationally deposited in the National Institute of Technology and Evaluation NITE Patent Microorganisms Depositary (NPMD, Address: Room 122, 2-5-8, Kazusakamatari, Kisarazu, Chiba, Japan 292-0818) in accordance with the Budapest Treaty under Accession Number: NITE BP-03536 (Receipt Number: NITE ABP-03536) (Date of receipt: September 14, 2021). NITE BP-03536 is a bacterium belonging to *Lactococcus lactis,* which is one of lactic acid bacteria. The bacteriological characteristics of NITE BP-03536 are shown in Table 1, Table 2, and Fig. 3 presented later.

NITE BP-03536 is present in fermented foods, and hence it is inferred to cause less impact on the environment in using for rearing an organism of fish and to be safe for humans. The above bacterium may be an isolated bacterium.

A composition for rearing an organism of fish according to an embodiment of the present invention contains bacterial cells or a cell culture product of NITE BP-03536, or an extract thereof. The bacterial cells may be cells isolated from a food or from the environment, or cultured one. The bacterial cells may be dead bacterial cells or living bacterial cells. The bacterial cells may be cells in a culture solution, a buffer solution, or the like, or in a product obtained by concentrating the culture solution, buffer solution, or the like to remove the liquid or a freeze-dried product thereof, or in a frozen stock.

The cell culture product may contain secretions, metabolites, and others from the bacterium. In the cell culture product, peptides, proteins, saccharides, enzymes, and organic acids produced by the bacterium, and medium (liquid medium or solid medium) containing any of them can be contained. The cell culture product may be the supernatant after culture of the bacterium. The culture supernatant can be obtained, for example, by removing the bacterium from the liquid medium in which the bacterium was cultured through centrifugation, filtration operations, or the like.

NITE BP-03536 can be cultured according to a common culture method for lactic acid bacteria. A representative culture method is, for example, a method of culturing with MRS (de Man, Rogosa and Sharpe) liquid medium or MRS agar medium at a temperature of 30°C.

The extract of the bacterial cells or the cell culture product is prepared in such a manner that the antibacterial activity of the bacterial cells or the cell culture product of the bacterium having a lacticin gene to *Lactococcus garvieae* is not lost. The extract can be obtained, for example, by treating the bacterial cells or the cell culture product of the bacterium through ultrasonic homogenization, bead grinding, freeze-thawing, or chemical dissolution. The extract may be obtained, for example, by performing salting-out, ultrafiltration, ion-exchange chromatography, or liquid-phase extraction with organic solvent on the bacterial cells or the cell culture product. These operations can be performed in combination, as appropriate. The extract can contain bacterial cell fragments, nucleic acids, peptides, proteins, saccharides, and enzymes from the bacterium. Herein, the bacterial cells or the cell culture product of the bacterium, or the extract thereof is also referred to as the "bacterial cell preparation".

Herein, the fish may be, but not limited to, the order Perciformes, the order Salmoniformes, the order Tetraodontiformes, the order Pleuronectiformes, the order Anguilliformes, the order Clupeiformes, the order Gadiformes, the order Scorpaeniformes, the order Cypriniformes, the order Clupeiformes, and the order Mugiliformes. The order Perciformes includes the family Carangidae, the family Scombridae, the family Lateolabracidae, the family Sparidae, the family Sebastidae, the family Triglidae, the family Hexagrammidae, the family Cottidae, the family Istiophoridae, the family Sphyraenidae, the family Trichiuridae, the family Latidae, the family Haemulidae, the family Oplegnathidae, the family Sillaginidae, the family Priacanthidae, the family Epinephelidae, the family Mullidae, the family Scaridae, and the family Labridae. The family Carangidae includes the genus *Seriola* (e.g., Japanese yellowtail, Great amberjack, Yellowtail amberjack, Almaco jack), the genus *Pseudocaranx* (e.g., striped jack), and the genus *Trachurus* (e.g., Japanese jack mackerel). The family Scombridae incudes the genus *Scomber* (e.g., chub mackerel, blue mackerel, Atlantic mackerel) and the genus *Thunnus* (e.g., Pacific bluefin tuna, Northern bluefin tuna, yellowfin tuna). The family Lateolabracidae includes the genus *Lateolabrax* (e.g., Japanese sea bass). The family Sparidae includes the genus *Rhabdosargus* (e.g., Goldlined seabream), the genus *Pagrus* (e.g., red seabream), and the genus *Evynnis* (e.g., crimson sea bream). The order Salmoniformes includes the family Salmonidae. The family Salmonidae includes the genus *Salmo* (e.g., rainbow trout, Atlantic salmon) and the genus *Oncorhynchus* (e.g., chum salmon, coho salmon). The order Tetraodontiformes includes the family Tetraodontidae and the family Monacanthidae. The family Monacanthidae includes the genus *Stephanolepis* (e.g., filefish) and the genus *Thamnaconus* (e.g., black scraper).

The compositions for rearing an organism of fish may be applied to an organism of fish. Examples of methods for applying any of the compositions for rearing an organism of fish to an organism of fish include methods of mixing water to rear an organism of fish and the composition for rearing an organism of fish. Examples of the methods of mixing water to rear an organism of fish and the composition for rearing an organism of fish include a method of dropping the composition for rearing an organism of fish into water, a method of pouring water into a rearing tank in which the composition for rearing an organism of fish is placed, and a method of adding the composition for rearing an organism of fish, dissolved in water, to water to rear an organism of fish. The composition for rearing an organism of fish may be automatically given to an organism of fish at constant intervals.

Each of the compositions for rearing an organism of fish may be a composition for oral administration. The composition for oral administration may be any composition to be orally administered to an organism of fish, without limitation. Each of the fish rearing composition may be a feed for fish (hereinafter, also referred to as a "fish feed") or a feed additive. The composition for oral administration may be added to an environment in which an organism of fish is reared (e.g., rearing water) and orally ingested by the organism of fish reared in the environment, into which lacticin or bacterial cell preparation of a bacterium having a lacticin gene is released by dissolution. The composition for oral administration to fish can inhibit infections caused by *Lactococcus garvieae* in an organism of fish to which the composition has been administered. The method for administering the composition for oral administration according to the present invention can give reduced burdens in administration as compared with conventional methods for inhibiting fish diseases, which involve fish-by-fish vaccine injection. In addition, the composition for oral administration according to the present invention is not limited to specific fish species, and applicable to rearing of a wide variety of biological species.

The fish feed may contain any component that is typically used for rearing or aquaculture of organisms of fish, and may be produced by any production method. An appropriate composition can be select for the fish feed, depending on the type and growth stage of the fish. The fish feed may contain a protein source such as squid meal, krill meal, fish meal, soybean meal, and corn gluten meal, and a binder such as gluten and starch. The fish feed may contain a known carrier or additive acceptable for feeds, and may contain, for example, a medicament for aquatic organisms such as an erythromycin preparation, an ampicillin preparation, a praziquantel preparation, a lysozyme chloride preparation, an oxytetracycline hydrochloride preparation, a spiramycin preparation, a sodium nifurstyrenate preparation, a lincomycin hydrochloride preparation, a flumequine preparation, and a glutathione preparation, a nutritional supplement substance such as a vitamin such as vitamin C, vitamin B 1, vitamin A, vitamin D, and vitamin E, and an amino acid such as lysine, methionine, and histidine, a pigment such as β-carotene, astaxanthin, and canthaxanthin, a mineral such as calcium and silicate, a trace metal, and a preservative.

The fish feed may have any shape and size, depending on the type and size or the like of the fish to be reared. For example, the fish feed may be a powdery feed obtained by mixing and pulverizing dry raw materials, a solidified feed (dry pellets) obtained by solidifying a powder, a pasty feed containing moisture (moist pellets), or a raw feed (slices of fish). In an example of methods for producing the fish feed, first, a common powdery feed for fish aquaculture, and lacticin or bacterial cell preparation of a bacterium having a lacticin gene are mixed together. This mixture is molded, for example, extrusion-molded by using a pasta machine or a syringe. Finally, the molded product is dried, for example, at 60°C to 65°C for about 2 hours, giving a fish feed containing lacticin or the bacterial cell preparation of a bacterium having a lacticin gene. Other examples of the fish feed include a feed obtained by dredging lacticin or the bacterial cell preparation of a bacterium having a lacticin gene over a common powdery feed for fish aquaculture, and a feed obtained by soaking a powdery feed for fish aquaculture in a liquid containing lacticin or the bacterial cell preparation of a bacterium having a lacticin gene (e.g., culture supernatant).

The amount of lacticin or the bacterial cell preparation of a bacterium having a lacticin gene contained in the fish feed is not limited as long as the antibacterial activity to *Lactococcus garvieae* is allowed to be exhibited, and may be an amount suitable for the bacterial cell density of *Lactococcus garvieae,* the rearing place, the rearing temperature, the oxygen concentration, the rearing density, the type of the fish of interest, and so on. The total amount of the bacterial cell preparation of a bacterium having a lacticin gene in the fish feed may be, for example, 1 × 10³ to 1 × 10¹² cfu (colony-forming unit)/g, 1 × 10⁴ to 1 × 10¹² cfu/g, or 1 × 10⁵ to 1 × 10¹² cfu/g. The colony-forming unit can be determined by an agar plate culture method. The total amount of lacticin and the bacterial cell preparation of a bacterium having a lacticin gene in the fish feed (content ratio) may be 0.001% by mass to 30% by mass, and preferably 0.01% by mass to 10% by mass to the feed weight. The total amount can be measured by weighing with a balance or the like.

The fish feed additive may be any additive that can be added to common fish feeds for aquaculture, without limitation. The feed additive may be liquid or powder, and may be freeze-dried. The feed additive may be lacticin or the bacterial cell preparation of a bacterium having a lacticin gene as it is. The feed additive may contain a liquid, a spreader, or the like for making it easy to allow lacticin or the bacterial cell preparation of a bacterium having a lacticin gene to be adhered to, absorbed in, or mixed with a fish feed. It is preferable that the feed additive be added to a feed in such a manner that the total amount of lacticin and the bacterial cell preparation of a bacterium having a lacticin gene contained in the feed falls within the range shown above.

The feed containing lacticin or the bacterial cell preparation of a bacterium having a lacticin gene may be fed once or multiple portions per day. The feed containing lacticin or the bacterial cell preparation of a bacterium having a lacticin gene may be fed once every several days. An appropriate period can be selected for feeding the feed containing lacticin or the bacterial cell preparation of a bacterium having a lacticin gene, for example, depending on the type of the fish of interest. The feed containing lacticin or the bacterial cell preparation of a bacterium having a lacticin gene may be continuously fed for the whole period of aquaculture (rearing), or fed only for part of the period. The "part of the period" may be, for example, 10% or more, 20% or more, 30% or more, 50% or more, 70% or more, or 90% or more of the whole period of rearing. In an aspect of the present embodiment, the upper limit value of the "part of the period" is not limited, and the part of the period may be, for example, less than 100% of the whole period of rearing, or 99% or less thereof. The period to feed the feed containing lacticin or the bacterial cell preparation of a bacterium having a lacticin gene may be, for example, 1 month to 3 years. The feed containing lacticin or the bacterial cell preparation of a bacterium having a lacticin gene may be fed in cycles of continuing feeding and suspending each for any period.

Each of the compositions for rearing an organism of fish may be a composition for soaking administration. The composition for soaking administration may be rearing water or a rearing water additive. The rearing water may be a liquid that is commonly used for rearing organisms of fish, and may be freshwater, brackish water, or seawater (including artificially prepared seawater). Rearing of an organism of fish may be performed in a natural environment, in an aquaculture pond, or in an aquarium. The rearing water may be aerated. The rearing water, which contains lacticin or the bacterial cell preparation of a bacterium having a lacticin gene, has antibacterial activity to *Lactococcus garvieae.* Organisms of fish soaked in the rearing water can be prevented from being affected by infections caused by *Lactococcus garvieae.* The composition for soaking administration can inhibit infections caused by *Lactococcus garvieae* in an organism of fish to which the composition has been administered. The method for soaking an organism of fish in the composition for soaking administration according to the present invention can give reduced burdens in administration as compared with conventional methods for inhibiting fish diseases, which involve fish-by-fish vaccine injection. The composition for soaking administration according to the present invention is not limited to specific fish species, and applicable to rearing of a wide variety of biological species.

The content of lacticin or the bacterial cell preparation of a bacterium having a lacticin gene in the rearing water is not limited as long as the antibacterial activity to *Lactococcus garvieae* is allowed to be exhibited, and may be an amount suitable for the bacterial cell density of *Lactococcus garvieae,* the rearing place, the rearing temperature, the oxygen concentration, the rearing density, the type of the fish of interest, and so on. The total amount of the bacterial cell preparation of a bacterium having a lacticin gene in the rearing water may be, for example, 1 × 10² to 1 × 10¹⁰ cfu/mL, 1 × 10³ to 1 × 10¹⁰ cfu/mL, or 1 × 10⁴ to 1 × 10¹⁰ cfu/mL. The colony-forming unit can be determined by an agar plate culture method. The total concentration of lacticin and the bacterial cell preparation of a bacterium having a lacticin gene contained in the rearing water is not limited, and may be 0.1 to 100,000 ppm, 1 to 100,000 ppm, or 10 to 100,000 ppm. The total amount of lacticin and the bacterial cell preparation of a bacterium having a lacticin gene can be measured by weighing with a balance or the like. The concentration can be calculated from the mass ratio to the rearing water on the basis of the total amount determined.

The rearing water additive may be an additive that can be added to common rearing water for organisms of fish, without limitation. The rearing water additive may be liquid or powder, and may be freeze-dried. The rearing water additive may be lacticin or the bacterial cell preparation of a bacterium having a lacticin gene as it is. It is preferable that the rearing water additive be added to rearing water in such a manner that the total amount of lacticin and the bacterial cell preparation of a bacterium having a lacticin gene contained in the rearing water falls within the range shown above.

The rearing water additive may be added to rearing water before the beginning of rearing of an organism of fish, or added to rearing water in which an organism of fish is reared. Examples of methods for adding the rearing water additive to rearing water include the aforementioned method of mixing water to rear an organism of fish and any of the compositions for rearing an organism of fish.

An appropriate period can be selected for soaking an organism of fish in the rearing water containing lacticin or the bacterial cell preparation of a bacterium having a lacticin gene. An organism of fish may be reared in the rearing water containing lacticin or the bacterial cell preparation of a bacterium having a lacticin gene continuously over the whole period to rear the organism of fish, or the fish may be soaked in the rearing water only for part of the period. The period to rear in the rearing water containing lacticin or the bacterial cell preparation of a bacterium having a lacticin gene may be, for example, 1 month to 3 years. Rearing in the rearing water containing lacticin or the bacterial cell preparation of a bacterium having a lacticin gene may be performed in cycles of continuing and suspending each for any period.

Each of the compositions for rearing an organism of fish may be a composition for injection administration (injection). The composition for injection administration can inhibit infections caused by *Lactococcus garvieae* in an organism of fish to which the composition has been administered.

The composition for injection administration may further contain, for example, any of buffers, isotonic agents, analgesic agents, antiseptic agents, antibacterial agents, and antioxidants. Examples of the buffers include phosphates, acetates, carbonates, and citrates. Examples of the isotonic agents include sodium chloride, glycerin, and D-mannitol. Examples of the analgesic agents include benzyl alcohol. Examples of agents for the purpose of antisepsis include thimerosal, p-hydroxybenzoates, phenoxyethanol, chlorobutanol, benzyl alcohol, phenethyl alcohol, dehydroacetic acid, sorbic acid, various antiseptic agents, antibiotics, and synthetic antibacterial agents. Examples of the antioxidants include sulfites and ascorbic acid. The composition for injection administration may contain any of photo-absorbing dyes to serve as an aid for storage and efficacy (e.g., riboflavin, adenine, adenosine), chelating agents and reductants for stabilization (e.g., vitamin C, citric acid), carbohydrates (e.g., sorbitol, lactose, mannitol, starch, sucrose, glucose, dextran), casein digests, and various vitamins.

The composition for injection administration can be obtained by adding lacticin or the bacterial cell preparation of a bacterium having a lacticin gene to a proper buffer. In an example, the composition for injection administration can be obtained by adding lacticin or the bacterial cell preparation of a bacterium having a lacticin gene to a conventional fish vaccine. The composition for injection administration may be the bacterial cell preparation of a bacterium having a lacticin gene as it is.

The amount of lacticin or the bacterial cell preparation of a bacterium having a lacticin gene contained in the composition for injection administration is not limited as long as the antibacterial activity to *Lactococcus garvieae* is allowed to be exhibited, and may be an amount suitable for the bacterial cell density of *Lactococcus garvieae,* the rearing place, the rearing temperature, the oxygen concentration, the rearing density, the type of the fish of interest, and so on. The total amount of the bacterial cell preparation of a bacterium having a lacticin gene in the composition for injection administration may be, for example, 1 × 10³ to 1 × 10¹² cfu (colony-forming unit)/g, 1 × 10⁴ to 1 × 10¹² cfu/g, or 1 × 10⁵ to 1 × 10¹² cfu/g. The total amount of lacticin and the bacterial cell preparation of a bacterium having a lacticin gene in the composition for injection administration may be 0.001% by mass to 30% by mass, and preferably 0.01% by mass to 10% by mass to the total weight of the composition for injection administration.

The composition for injection administration may be administered to an organism of fish once or multiple times. For example, the composition for injection administration is intramuscularly or intraperitoneally administered to an organism of fish. The dose in one administration may be 0.05 mL to 3.0 mL. The time of administration may be the juvenile stage, young stage, or adult stage of the fish.

An embodiment of the present invention is use of at least one selected from the group consisting of lacticin, bacterial cells or a cell culture product of a bacterium having a lacticin gene, and an extract of the bacterial cells or the cell culture product of the bacterium having the lacticin gene in production of the composition for rearing an organism of fish.

### [Methods for rearing organism of fish]

A method for rearing an organism of fish according to an embodiment of the present invention includes applying at least one selected from the group consisting of lacticin, bacterial cells or a cell culture product of a bacterium having a lacticin gene, and an extract of the bacterial cells or the cell culture product of the bacterium having the lacticin gene. Each applying may be oral administration, soaking administration, or injection administration.

A method for rearing an organism of fish according to an embodiment of the present invention includes allowing an organism of fish to ingest at least one selected from the group consisting of lacticin, bacterial cells or a cell culture product of a bacterium having a lacticin gene, and an extract of the bacterial cells or the cell culture product of the bacterium having the lacticin gene. Lacticin and the bacterial cell preparation of a bacterium having a lacticin gene may be provided as any of the above compositions for rearing an organism of fish.

A method for rearing an organism of fish according to an embodiment of the present invention includes soaking an organism of fish in rearing water containing at least one selected from the group consisting of lacticin, bacterial cells or a cell culture product of a bacterium having a lacticin gene, and an extract of the bacterial cells or the cell culture product of the bacterium having the lacticin gene. The rearing water containing lacticin and the bacterial cell preparation of a bacterium having a lacticin gene may be provided as any of the above compositions for rearing an organism of fish.

In the methods for rearing an organism of fish, any of the compositions for rearing an organism of fish may be given according to the aforementioned method of mixing water to rear an organism of fish and the composition for rearing an organism of fish.

A method for rearing an organism of fish according to an embodiment of the present invention includes injecting lacticin, bacterial cells or a cell culture product of a bacterium having a lacticin gene, and an extract of the bacterial cells or the cell culture product of the bacterium having the lacticin gene into an organism of fish. Lacticin and the bacterial cell preparation of a bacterium having a lacticin gene may be provided as any of the above compositions for rearing an organism of fish.

An embodiment of the present invention is use of lacticin, bacterial cells or a cell culture product of a bacterium having a lacticin gene, and an extract of the bacterial cells or the cell culture product of the bacterium having the lacticin gene in rearing an organism of fish.

### [Compositions for preventing or treating disease caused by Lactococcus garvieae in fish]

A composition for preventing or treating a disease caused by *Lactococcus garvieae* (streptococci) in fish according to an embodiment of the present invention contains at least one selected from the group consisting of lacticin, bacterial cells or a cell culture product of a bacterium having a lacticin gene, and an extract of the bacterial cells or the cell culture product of the bacterium having the lacticin gene. Fish infections caused by *Lactococcus garvieae* can be prevented or treated by using lacticin or the bacterial cell preparation of a bacterium having a lacticin gene. Herein, treatment includes mitigation of a symptom and improvement in and complete cure of a symptom. Each of the preventive or therapeutic compositions may be a medicament for animals.

Lacticin and the bacterial cell preparation of a bacterium having a lacticin gene each exhibit antibacterial activity to *Lactococcus garvieae,* and are capable of inhibiting the growth of *Lactococcus garvieae* or killing *Lactococcus garvieae.* Lacticin and the bacterial cell preparation of a bacterium having a lacticin gene each exhibit antibacterial activity not only to previous (type I) *Lactococcus garvieae,* but also to novel (type II) *Lactococcus garvieae.* Lacticin and the bacterial cell preparation of a bacterium having a lacticin gene each have direct antibacterial activity to causal bacteria for infections, with the mechanism for inhibiting infections being independent of hosts. Therefore, lacticin and the bacterial cell preparation of a bacterium having a lacticin gene are capable of preventing fish infections in a more universal manner, irrespective of the type or growth stage of the fish of interest. Use of lacticin or the bacterial cell preparation of a bacterium having a lacticin gene enables more stable aquaculture of organisms of fish.

Each of the preventive or therapeutic compositions may be a composition for oral administration, and may be administered in the form of a feed or feed additive to an organism of fish. The preventive or therapeutic composition may contain a component that can be contained in the composition for oral administration to be used for rearing an organism of fish. The preventive or therapeutic composition may further contain a known component having antibacterial activity to *Lactococcus garvieae* or component that increases the immunoreactivity of fish. The same shapes, production methods, and administration methods as for the composition for oral administration to be used for rearing an organism of fish may be applied to the preventive or therapeutic composition. The content of lacticin or the bacterial cell preparation of a bacterium having a lacticin gene in the preventive or therapeutic composition is not limited as long as the content is such that infections caused by *Lactococcus garvieae* can be prevented or treated in an organism of fish to which the composition has been administered, and may be in the same content range as in the composition for oral administration to be used for rearing an organism of fish.

Each of the preventive or therapeutic compositions may be a composition for soaking administration, and may be in the form of rearing water or a rearing water additive. The preventive or therapeutic composition as rearing water or a rearing water additive may be used or added in the same manner as rearing water or rearing water additives to be used for rearing organisms of fish. The content of lacticin or the bacterial cell preparation of a bacterium having a lacticin gene in the preventive or therapeutic composition is not limited as long as the content is such that infections caused by *Lactococcus garvieae* can be prevented or treated in an organism of fish which has been soaked in the composition, and may be in the same content range as in the rearing water to be used for rearing an organism of fish.

Each of the preventive or therapeutic compositions may be a composition for injection administration (injection). The preventive or therapeutic composition may contain a component that can be contained in the composition for injection administration to be used for rearing an organisms of fish. The preventive or therapeutic composition may further contain a known component having antibacterial activity to *Lactococcus garvieae* or component that increases the immunoreactivity of fish. The same shapes, production methods, and administration methods as for the composition for injection administration to be used for rearing an organism of fish may be applied to the preventive or therapeutic composition. The content of lacticin or the bacterial cell preparation of a bacterium having a lacticin gene in the preventive or therapeutic composition is not limited as long as the content is such that infections caused by *Lactococcus garvieae* can be prevented or treated in an organism of fish to which the composition has been administered by injection, and may be in the same content range as in the composition for injection administration to be used for rearing an organism of fish.

An embodiment of the present invention is use of at least one selected from the group consisting of lacticin, bacterial cells or a cell culture product of a bacterium having a lacticin gene, and an extract of the bacterial cells or the cell culture product of the bacterium having the lacticin gene in production of a composition for preventing or treating a fish infection caused by *Lactococcus garvieae.*

### [Methods for preventing or treating disease caused by Lactococcus garvieae in fish]

A method for preventing or treating a disease caused by *Lactococcus garvieae* in fish according to an embodiment of the present invention includes applying at least one selected from the group consisting of lacticin, bacterial cells or a cell culture product of a bacterium having a lacticin gene, and an extract of the bacterial cells or the cell culture product of the bacterium having the lacticin gene to an organism of fish. Each applying may be oral administration, soaking administration, or injection administration.

A method for preventing or treating a disease caused by *Lactococcus garvieae* in fish according to an embodiment of the present invention includes allowing an organism of fish to ingest at least one selected from the group consisting of lacticin, bacterial cells or a cell culture product of a bacterium having a lacticin gene, and an extract of the bacterial cells or the cell culture product of the bacterium having the lacticin gene. Lacticin and the bacterial cell preparation of a bacterium having a lacticin gene may be provided as any of the preventive or therapeutic compositions to an organism of fish.

A method for preventing or treating a disease caused by *Lactococcus garvieae* in fish according to an embodiment of the present invention includes soaking an organism of fish in rearing water containing at least one selected from the group consisting of lacticin, bacterial cells or a cell culture product of a bacterium having a lacticin gene, and an extract of the bacterial cells or the cell culture product of the bacterium having the lacticin gene. The rearing water containing lacticin and the bacterial cell preparation of a bacterium having a lacticin gene may be provided as any of the preventive or therapeutic compositions.

A method for preventing or treating a disease caused by *Lactococcus garvieae* in fish according to an embodiment of the present invention includes injecting at least one selected from the group consisting of lacticin, bacterial cells or a cell culture product of a bacterium having a lacticin gene, and an extract of the bacterial cells or the cell culture product of the bacterium having the lacticin gene into an organism of fish. Lacticin and the bacterial cell preparation of a bacterium having a lacticin gene may be provided as any of the preventive or therapeutic compositions to an organism of fish.

An embodiment of the present invention is use of at least one selected from the group consisting of lacticin, bacterial cells or a cell culture product of a bacterium having a lacticin gene, and an extract of the bacterial cells or the cell culture product of the bacterium having the lacticin gene for preventing or treating a disease caused by *Lactococcus garvieae* in fish.

### [Growth inhibitor or bactericide, and growth-inhibiting or bactericidal method for Lactococcus garvieae]

A growth inhibitor or a bactericide for *Lactococcus garvieae* according to an embodiment of the present invention contains lacticin. A growth inhibitor or a bactericide for *Lactococcus garvieae* according to an embodiment of the present invention contains bacterial cells or a cell culture product of a bacterium having a lacticin gene, or an extract thereof. Lacticin and the bacterial cell preparation of a bacterium having a lacticin gene are each capable of inhibiting the growth of *Lactococcus garvieae* or killing *Lactococcus garvieae.* The growth inhibitor or the bactericide may be in the same form as the compositions for rearing an organism of fish. The growth inhibitor or the bactericide may further contain a substance having antibacterial activity to *Lactococcus garvieae.*

The content of the bacterial cell preparation of a bacterium having a lacticin gene contained in the growth inhibitor or the bactericide for *Lactococcus garvieae* is not limited, and may be, for example, 1 × 10³ to 1 × 10¹² cfu/mL, 1 × 10⁵ to 1 × 10¹² cfu/mL, or 1 × 10⁷ to 1 × 10¹² cfu/mL. The total concentration of lacticin and the bacterial cell preparation of a bacterium having a lacticin gene contained in the growth inhibitor or the bactericide for *Lactococcus garvieae* is not limited, and may be 0.01 to 100,000 ppm, 0.1 to 100,000 ppm, or 1 to 100,000 ppm.

A growth-inhibiting or bactericidal method for *Lactococcus garvieae* according to an embodiment of the present invention includes bringing at least one selected from the group consisting of lacticin, bacterial cells or a cell culture product of a bacterium having a lacticin gene, and an extract of the bacterial cells or the cell culture product of the bacterium having the lacticin gene into contact with *Lactococcus garvieae.* Any method of bringing lacticin or the bacterial cell preparation of a bacterium having a lacticin gene into contact with *Lactococcus garvieae* is applicable without limitation. For example, lacticin or the bacterial cell preparation of a bacterium having a lacticin gene may be added to water in which *Lactococcus garvieae* is present, and an object with the presence of *Lactococcus garvieae* may be soaked in a liquid containing lacticin or the bacterial cell preparation of a bacterium having a lacticin gene. A host infected with *Lactococcus garvieae* may be allowed to ingest a composition containing lacticin or the bacterial cell preparation of a bacterium having a lacticin gene, and a host infected with *Lactococcus garvieae* may be soaked in rearing water containing lacticin or the bacterial cell preparation of a bacterium having a lacticin gene. A composition containing lacticin or the bacterial cell preparation of a bacterium having a lacticin gene may be injected into a host infected with *Lactococcus garvieae.* Lacticin and the bacterial cell preparation of a bacterium having a lacticin gene may be used in the form of any of the growth inhibitor or the bactericide for *Lactococcus garvieae.*

An embodiment of the present invention is use of at least one selected from the group consisting of lacticin, bacterial cells or a cell culture product of a bacterium having a lacticin gene, and an extract of the bacterial cells or the cell culture product of the bacterium having the lacticin gene in production of a growth inhibitor or a bactericide for *Lactococcus garvieae.*

An embodiment of the present invention is use of at least one selected from the group consisting of lacticin, bacterial cells or a cell culture product of a bacterium having a lacticin gene, and an extract of the bacterial cells or the cell culture product of the bacterium having the lacticin gene for inhibiting the growth of or killing *Lactococcus garvieae.*

### EXAMPLES

Hereinafter, the present invention will be described in more detail with reference to Examples, but the present invention is not limited to those Examples.

### [Experiment 1: Isolation and identification of NITE BP-03536]

A source for isolation (pickled chives) was ground together with sterile water. The solution given by grinding was appropriately diluted and added to 1/2 MRS liquid medium, and subjected to enrichment culture. The enrichment culture solution was smeared on MRS agar medium containing calcium carbonate, and a microorganism that formed a halo was isolated. Hereinafter, this isolate is referred to as isolate A. No bubble was generated when the culture solution of isolate A was suspended in hydrogen peroxide. From the absence of catalase, isolate A was confirmed to be a lactic acid bacterium.

Isolate A was identified through 16S rRNA gene analysis, morphological observation, and physiological/biochemical characteristics test.

### (1) 16S rRNA gene analysis

Genomic DNA was extracted from isolate A, and PCR amplification of a 16S rRNA gene was performed by using the extracted genomic DNA as a template with a forward primer for cloning, 9F, and a reverse primer for cloning, 1510R (NAKAGAWA Yasuyoshi et al.: Gene Analysis Method - Nucleotide Sequence Determination Method for 16S rRNA Gene, edited by The Society for Actinomycetes Japan, Identification Manual of Actinomycetes, pp. 88-117, Business Center for Academic Societies Japan, 2001). Tks Gflex DNA polymerase (manufactured by Takara Bio Inc.) was used in the PCR amplification performed, and a PCR amplification product was purified.

Cycle sequence reaction was performed with the purified PCR amplification product. A BigDye Terminator v3.1 Cycle Sequencing Kit was used in the cycle sequence reaction performed. The resulting reaction solution was purified, and the purified solution was subjected to DNA sequence analysis (3130xl DNA Analyzer) to determine the nucleotide sequence of the 16S rRNA gene of the template DNA extracted from isolate A. Primers used for the sequence analysis were 9F, 515F, 1099F, 536R, 926R, and 1510R (NAKAGAWA Yasuyoshi et al.: Gene Analysis Method - Nucleotide Sequence Determination Method for 16S rRNA Gene, edited by The Society for Actinomycetes Japan, Identification Manual of Actinomycetes, pp. 88-117, Business Center for Academic Societies Japan, 2001).

With the microorganism identification system "ENKI" (manufactured by TechnoSuruga Laboratory Co., Ltd.), the nucleotide sequence of the 16S rRNA gene of isolate A was subjected to BLAST homology search for the microorganism identification database DB-BA15.0 (established by TechnoSuruga Laboratory Co., Ltd.) and international nucleotide sequence databases (DDBJ/ENA(EMBL)/GenBank). The nucleotide sequence of the 16S rRNA gene of isolate A exhibited 99.93% identity with the nucleotide sequence of the 16S rRNA gene of *Lactococcus lactis* subsp. *lactis* (NCDO604; Accession No. AB 100803), 99.93% identity with the nucleotide sequence of the 16S rRNA gene of *Lactococcus lactis* subsp. *hordniae* (NCDO2181), 99.46% identity with the nucleotide sequence of the 16S rRNA gene of *Lactococcus lactis* subsp. *tructae* (L105), and 99.39% identity with the nucleotide sequence of the 16S rRNA gene of *Lactococcus lactis* subsp. *cremoris* (NCDO607).

### (2) Morphological observation and physiological/biochemical characteristics test

Isolate A was applied onto MRS agar medium, and subjected to aerobic culture at a temperature of 30°C for 48 hours, and the cell morphology, Gram stainability, mobility, and colony morphology were observed by the following methods. The cell morphology was observed with the optical microscope BX50F4 (manufactured by Olympus Corporation). In the Gram staining, Favor G "NISSUI" (manufactured by Nissui Pharmaceutical Co., Ltd.) was used. The colony morphology was observed with the stereomicroscope SMZ800N (manufactured by Nikon Corporation). According to methods described in Barrow & Feltham (Cowan and Steel's Manual for the Identification of Medical Bacteria, 3rd ed. Cambridge: Cambridge University Press; 1993.), test was performed on catalase reaction, oxidase reaction, acid/gas production from glucose, and oxidation/fermentation (O/F) of glucose. The physiological/biochemical characteristics reaction of the bacterium was examined by using an API50CHB kit (manufactured by bioMerieux, France).

As shown in Fig. 3(A), isolate A formed circular colonies. As shown in Fig. 3(B), isolate A was positive for Gram stainability. Table 1 and Table 2 show results of the physiological/biochemical characteristics test and fermentability test for isolate A. Isolate A was Gram-positive, ovoid in shape without mobility, formed no spore, exhibited negativity for catalase reaction and oxidase reaction, and fermented glucose. These characteristics were consistent with those of the genus *Lactococcus,* to which isolate A was expected to belong as shown by the results of the 16S rDNA partial nucleotide sequence analysis. Results of the fermentability test performed with an API kit showed that isolate A fermented ribose, D-xylose, galactose, mannitol, maltose, etc., and did not ferment melibiose, melicitose, etc. Isolate A exhibited arginine dihydrolase activity. Isolate A grew at 15°C, and grew in the presence of 4% NaCl. From these characteristics, isolate A was found to be a novel isolate belonging to *Lactococcus lactis* subsp. *lactis.* Isolate A was internationally deposited as NITE BP-03536 (Receipt Number: NITE ABP-03536).

As a result of sequence analysis of the NITE BP-03536 genomic DNA, NITE BP-03536 had a lacticin Q gene known as an antibacterial peptide. The amino acid sequence of the lacticin Q gene possessed by NITE BP-03536 and the corresponding DNA sequence are shown in SEQ ID NOs: 1 and 2.

**[Table 1]**

| Test item \| | | NITE BP-03536 |
|---|---|---|
| Culture temperature | | 30°C |
| Cell morphology | | oval coccus (0.6-0.8×1.0-1.3 µm) |
| Gram stainability | | + |
| Presence or absence of spore | | - |
| Mobility | | - |
| Colony morphology | Medium | MRS agar medium |
| | Culture time | 72 hours |
| | Diameter | 1 .0-2.0 mm |
| | Color | cream |
| | Shape | circular |
| | Elevation | lens-like |
| | Margin | entire |
| | Surface shape, etc. | smooth |
| | Transparency | opaque |
| | Viscosity | butter-like |
| Growth temperature test | 15°C | + |
| | 37°C | + |
| | 45°C | - |
| Growth in the presence of 2% NaCl | | + |
| Growth in the presence of 4% NaCl | | + |
| Arginine dihvdrolase activity | | + |
| Catalase reaction | | - |
| Oxidase reaction | | - |
| Acid/gas production from glucose (acid production/gas production) | | +/+ |
| O/F test (oxidation/fermentation) | | NT |

| | | |
|---|---|---|
| + : positive, - : negative, NT: Not test | | |

**[Table 2]**

| Item | Substrate component | Result |
|---|---|---|
| 0 | Control | - |
| 1 | Glycerol | - |
| 2 | Erythritol | - |
| 3 | D-Arabinose | - |
| 4 | L-Arabinose | + |
| 5 | Ribose | + |
| 6 | D-Xylose | + |
| 7 | L-Xylose | - |
| 8 | Adonitol | - |
| 9 | β-Methyl-D-xylose | - |
| 10 | Galactose | + |
| 11 | Glucose | + |
| 12 | Fructose | + |
| 13 | Mannose | + |
| 14 | Sorbose | - |
| 15 | Rhamnose | - |
| 16 | Dulcitol | - |
| 17 | Inositol | - |
| 18 | Mannitol | + |
| 19 | Sorbitol | - |
| 20 | α-Methyl-D-mannoside | - |
| 21 | α-Methyl-D-glucoside | - |
| 22 | N-Acetylglucosamine | + |
| 23 | Amyqdalin | + |
| 24 | Arbutin | + |
| 25 | Esculin | + |
| 26 | Salicin | + |
| 27 | Cellobiose | + |
| 28 | Maltose | + |
| 29 | Lactose | + |
| 30 | Melibiose | - |
| 31 | Sucrose | + |
| 32 | Trehalose | + |
| 33 | Inulin | - |
| 34 | Melicitose | - |
| 35 | Raffinose | - |
| 36 | Starch | + |
| 37 | Glycogen | - |
| 38 | Xylitol | - |
| 39 | Gentiobiose | + |
| 40 | D-Turanose | - |
| 41 | D-Lyxose | - |
| 42 | D-Tagatose | - |
| 43 | D-Fucose | - |
| 44 | L-Fucose | - |
| 45 | D-Arabitol | - |
| 46 | L-Arabitol | - |
| 47 | Gluconate | + |
| 48 | 2-Ketogluconate | - |
| 49 | 5-Ketogluconate | - |

### [Experiment 2: Antibacterial activity test of bacterial cell preparation of a bacterium having a lacticin gene]

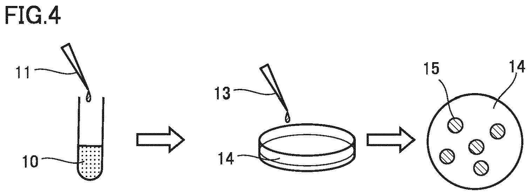

Examination was performed on whether bacterial cell preparations of NITE BP-03536 inhibits the growth of *Lactococcus garvieae* type I (TUMSAT-K9408 strain) and type II (TUMSAT-LgN1 strain). With reference to Fig. 4, the experimental method will be described. To 270 µL of Todd-Hewitt (TH) medium, 3 µL of *Lactococcus garvieae* (approximately 10⁵ cfu/mL) was added to prepare a bacterial solution 10 containing *Lactococcus garvieae.* To this bacterial solution 10, 30 µL of a bacterial cell preparation 11 of NITE BP-03536 was added, and shaking culture was performed at 25°C for 8 hours. To a TH agar medium 14, 5 µL of a bacterial culture solution 13 was dropped, culture was performed at 25°C for 12 hours, and whether a colony 15 of *Lactococcus garvieae* formed was observed. If the number of colonies observed was five or less, it was determined that there was antibacterial activity. The bacterial cell preparations of NITE BP-03536 used in the experiments were prepared in the following manner. First, NITE BP-03536 was cultured in MRS Broth at a temperature of 30°C, giving culture solutions. Thereafter, the culture solutions were centrifuged to precipitate bacterial cells, and the culture supernatants were collected. The culture supernatants collected were 10-fold concentrated by using an ultrafiltration membrane (Merck Millipore, AMICON ULTRA-15 3 KDa cutoff), giving bacterial cell preparations. As a negative control, in place of bacterial cell preparations 11 of NITE BP-03536, a medium for lactic acid bacteria (MRS Broth) was added to bacterial solution 10 containing *Lactococcus garvieae.* The results of the antibacterial activity test are shown in Table 3.

**[Table 3]**

| | Lactococcus garvieae type I | Lactococcus garvieae type II |
|---|---|---|
| NITE BP-03536 | + | + |
| control | - | - |

| | | |
|---|---|---|
| +:presence of antibacterial activity, -:absence of antibacterial activity | | |

The culture supernatants of NITE BP-03536 having a lacticin gene inhibited the growth of *Lactococcus garvieae* type I and type II. It was demonstrated that the bacterial cell preparations of a bacterium having a lacticin gene have antibacterial activity to *Lactococcus garvieae.*

### [Experiment 3: Examination of preventive or therapeutic effect of oral administration of bacterial cell preparation of a bacterium having a lacticin gene for streptococcosis in fish]

Examination was performed on whether oral administration of a bacterial cell preparation of NITE BP-03536 reduces the death caused by streptococcosis in Japanese yellowtail. Juvenile fish (mojako) of Japanese yellowtail were distributed to two test sections having 25 fish per section, and the following feeds were given.
(1) Control: common feed (extruded pellet (EP) feed for growing Japanese yellowtail from FEED ONE CO., LTD.)
(2) Feed soaked in culture supernatant of NITE BP-03536 (0.3 mL/g of feed)

The culture supernatants of NITE BP-03536 were prepared in the following method. First, the lactic acid bacterium (NITE BP-03536) was cultured in MRS Broth at a temperature of 30°C, giving culture solutions. Thereafter, the culture solutions were centrifuged to precipitate bacterial cells, and the culture supernatants were collected.

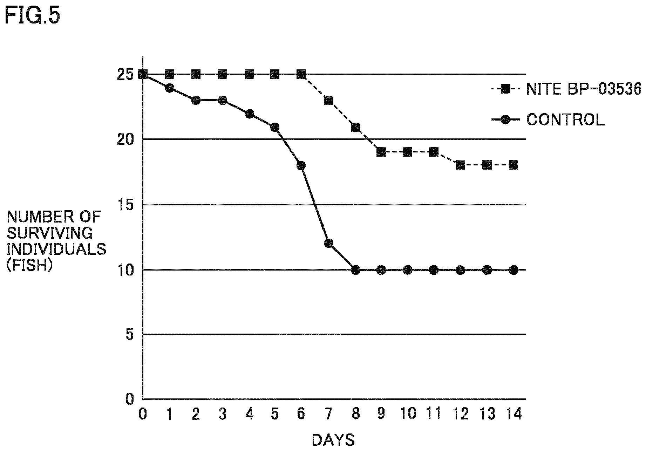

Juvenile fish of Japanese yellowtail were grown for habituation for approximately 1 week in an aquarium in which artificial seawater (manufactured by Tomita Pharmaceutical Co., Ltd.) was circulated, and then the test was initiated. The temperature of the rearing water was 23 to 25°C. The estimated body weight of the juvenile fish of Japanese yellowtail at the initiation of the test was approximately 20 g. The fish were satiated with the feeds once per day. Two weeks after the initiation of providing, the Japanese yellowtail were soaked in rearing water containing 1 × 10⁸ cfu/mL of *Lactococcus garvieae* type II (18-15 strain) for 1 hour to infect the fish with *Lactococcus garvieae.* After the infection, the Japanese yellowtail were returned to the former aquariums, and rearing was further continued for 2 weeks with administration of the feeds. The number of surviving Japanese yellowtails after the test is shown in Table 4 and Fig. 5. In Fig. 5, the ordinate indicates the number of surviving Japanese yellowtails infected with *Lactococcus garvieae,* and the abscissa indicates the number of rearing days after infection with *Lactococcus garvieae.*

**[Table 4]**

| | The number of surviving individuals (fish) | Survival rate |
|---|---|---|
| NITE BP-03536 | 18 | 72% |
| control | 10 | 40% |

The survival rate of Japanese yellowtails to which the bacterial cell preparation of NITE BP-03536 was orally administered increased as compared with the control. It was demonstrated that oral administration of a bacterial cell preparation of a bacterium having a lacticin gene can reduce the death caused by streptococcosis in an organism of fish. It was also demonstrated that infection with *Lactococcus garvieae* in organisms of fish was inhibited through oral administration of a bacterial cell preparation of a bacterium having a lacticin gene.

### REFERENCE SIGNS LIST

10 Bacterial solution containing *Lactococcus garvieae;* 11 Bacterial cell preparation of NITE BP-03536; 13 Bacterial culture solution; 14 TH agar medium; 15 Colony.

## Claims

1. A composition for rearing an organism of fish, comprising at least one selected from the group consisting of lacticin, bacterial cells or a cell culture product of a bacterium having a lacticin gene, and an extract of the bacterial cells or the cell culture product.

2. A composition for preventing or treating a disease caused by *Lactococcus garvieae* in fish, comprising at least one selected from the group consisting of lacticin, bacterial cells or a cell culture product of a bacterium having a lacticin gene, and an extract of the bacterial cells or the cell culture product.

3. The composition according to claim 1 or 2, wherein the lacticin is a peptide containing any sequence of (a) to (c) and having antibacterial activity:
(a) an amino acid sequence set forth in SEQ ID NO: 1,
(b) an amino acid sequence having 69% or more identity with an amino acid sequence set forth in SEQ ID NO: 1, and
(c) an amino acid sequence formed by deletion, substitution, insertion, and/or addition of 1 or more and 16 or less amino acid residues in an amino acid sequence set forth in SEQ ID NO: 1.

4. The composition according to claim 1 or 2, wherein the lacticin is at least one selected from lacticin Q and lacticin Z, and the lacticin gene is at least one selected from a lacticin Q gene and a lacticin Z gene.

5. The composition according to claim 1 or 2, wherein the bacterium having the lacticin gene is NITE BP-03536.

6. The composition according to claim 1 or 2, wherein the fish belongs to the order Perciformes or the order Salmoniformes.

7. The composition according to claim 1 or 2, wherein the composition is a feed, a feed additive, rearing water, a rearing water additive, or an injection.

8. A method for rearing an organism of fish, comprising applying the composition according to claim 1 to the organism of fish.

9. A method for preventing or treating a disease caused by *Lactococcus garvieae* in fish, comprising applying the composition according to claim 2 to the organism of fish.

10. The method according to claim 8 or 9, wherein the applying is oral administration, soaking administration, or injection administration.

11. The method according to claim 8 or 9, wherein the fish belongs to the order Perciformes or the order Salmoniformes.

12. A growth inhibitor or a bactericide for *Lactococcus garvieae,* comprising at least one selected from the group consisting of lacticin, bacterial cells or a cell culture product of a bacterium having a lacticin gene, and an extract of the bacterial cells or the cell culture product.

13. A growth-inhibiting or bactericidal method for *Lactococcus garvieae,* comprising bringing at least one selected from the group consisting of lacticin, bacterial cells or a cell culture product of a bacterium having a lacticin gene, and an extract of the bacterial cells or the cell culture product into contact with *Lactococcus garvieae.*

14. A bacterium of Accession Number: NITE BP-03536.

15. Bacterial cells or a bacterial cell culture of the bacterium according to claim 14, or an extract thereof.
